Europäisches Patentamt

European Patent Office

Office européen des brevets

(11) Veröffentlichungsnummer : **0 121 085**
**B1**

(12) **EUROPÄISCHE PATENTSCHRIFT**

(45) Veröffentlichungstag der Patentschrift :
04.03.87

(51) Int. Cl.⁴ : **A 61 M 1/14, A 61 M 5/14**

(21) Anmeldenummer : **84101994.6**

(22) Anmeldetag : **25.02.84**

(54) **Gerät zur Aufbereitung medizinischer Infusionslösungen.**

(30) Priorität : 01.03.83 DE 3307112

(43) Veröffentlichungstag der Anmeldung :
10.10.84 Patentblatt 84/41

(45) Bekanntmachung des Hinweises auf die Patenterteilung : 04.03.87 Patentblatt 87/10

(84) Benannte Vertragsstaaten :
AT CH DE FR IT LI NL SE

(56) Entgegenhaltungen :
EP-A- 0 004 600
EP-A- 0 042 939
EP-A- 0 062 148
DE-A- 2 734 075
DE-A- 3 110 022
DE-A- 3 121 098
DE-A- 3 122 756
GB-A- 2 091 126
US-A- 4 153 554
US-A- 4 338 190
US-A- 4 370 983

(73) Patentinhaber : Sartorius GmbH.
Weender Landstrasse 94-108
D-3400 Göttingen (DE)

(72) Erfinder : Knothe, Erich
Hasenwinkel 4
D-3406 Bovenden 1 (DE)
Erfinder : Köhn, Heinz Gerhard, Dr.
Grüner Weg 6
D-3402 Dransfeld (DE)
Erfinder : Pradel, Günter
Söhlwiese 21
D-3400 Göttingen (DE)

(74) Vertreter : Köhler, Rudolf
c/o Sartorius GmbH Weender Landstrasse 94-108
D-3400 Göttingen (DE)

**Beschreibung**

Die Erfindung betrifft ein Gerät zur Aufbereitung medizinischer Infusionslösungen. Neben der Haemodialyse hat sich in den letzten Jahren die Haemofiltration als Behandlungsmethode bei akutem und chronischem Nierenversagen immer mehr durchgesetzt. Zum Stand der Technik wird hingewiesen auf GB-A-1 563 840, US-A-3 579 441, GB-A-1 366 086, GB-A-1 555 389, US-A-4 219 422. Dabei werden dem Patienten durch Filtration die sich im Blut angehäuften Schadstoffe und ein erhebliches Maß Blutflüssigkeit entzogen. Die Schadstoffe und Flüssigkeit werden verworfen. Zumindest ein Teil der Flüssigkeit muß dem Patienten in Form von Infusionslösungen wieder zugeführt werden. Diese Infusionslösungen werden zum direkten Verbrauch in handelsüblichen Gebinden, steril verpackt und sterilisiert geliefert und direkt über die Dialysemaschinen oder Haemofiltrationsmaschinen dem Patienten während der Behandlung zugeführt. Wegen der erheblichen Mengen, die jeder Patient benötigt, ist diese Handelsform der Infusionslösung unter wirtschaftlichen Gesichtspunkten unbefriedigend. Hinzu kommt, daß durch längere Lagerung die Gefahr einer Verkeimung der Lösung besteht und damit der Patient einem höheren Risiko ausgesetzt ist. Da in Kliniken und Dialysezentren eine größere Anzahl solcher « künstlicher Nieren » vorhanden sind, ist daher der Bedarf an Infusionslösung sehr groß. Auch bei der medizinischen Behandlung nierenerkrankter Patienten nach dem Prinzip der Dialyse, besonders aber bei der Peritonealdialyse, werden ebenfalls keimfreie, unter medizinischen Gesichtspunkten besonders zusammengesetzte Infusionslösungen bzw. Behandlungslösungen benötigt.

Andererseits sind auch bereits Anlagen zur direkten Herstellung der Infusionslösung aus den einzelnen Bestandteilen bekannt, z. B. EP-A1-0 042 939, die jedoch für den klinischen Betrieb zu ungenau arbeiten. Hinzu kommt, daß die bekannten Geräte keine Einrichtung besitzen, um eine Verkeimung der im Gerät aufbereiteten Infusionslösung zu verhindern, wenn keine permanente Entnahme der Infusionslösung erfolgt, sondern längere Entnahmepausen zu berücksichtigen sind.

Der Erfindung liegt daher die Aufgabe zugrunde, ein Gerät zu schaffen, welches in der Bedienung einfach und sicher aufgebaut ist und die Möglichkeit zuläßt, Infusionslösungen zur unmittelbaren Verwendung am Patienten in gewünschter exakter Konzentration und Zusammensetzung herzustellen und das eine Verkeimung der Infusionslösung im Gerät während längerer Entnahmepausen ausschließt.

Diese Aufgabe wird durch die im Anspruch 1 angegebenen Merkmale gelöst. Vorteilhafte Weiterbildungen sind in den Unteransprüchen angegeben. Hierbei wurde geräteseitig berücksichtigt, daß in allen Kliniken und Dialysestationen bereits großtechnische Anlagen vorhanden sind, mit denen Reinstwasser auf einer zentralen, nach dem Prinzip der Umkehrosmose arbeitenden Wasserversorgungsanlage herstellbar und somit das für die Herstellung der Infusionslösung notwendige Reinstwasser in großer Menge vorrätig ist. Der bisher in den handelsüblichen Gebinden von fertiger Infusionslösung vorhandene Anteil an Reinstwasser braucht also nicht mehr gelagert und transportiert zu werden.

Das erfindungsgemäße Gerät kann daher portabel ausgebildet sein und die Lagerhaltung beschränkt sich nur noch auf die relativ kleinen Mengen an Elektrolytkonzentrat zur Herstellung der Infusionslösung. Die auf dem Gerät hergestellte Infusionslösung kann daher praktisch kurz nach ihrer Herstellung In-Line dem Patienten über die Dialysemaschine oder die Haemofiltrationsmaschine zugeführt werden. Die zugeführte Lösung ist pyrogenfrei und keimfrei, auch wenn die dauernde Entnahme durch längere Entnahmepause (Nichtentnahme von Infusionslösung) unterbrochen war.

Ein bevorzugtes Ausführungsbeispiel des Gerätes ist in der beiliegenden Zeichnung näher erläutert. Dabei zeigt :

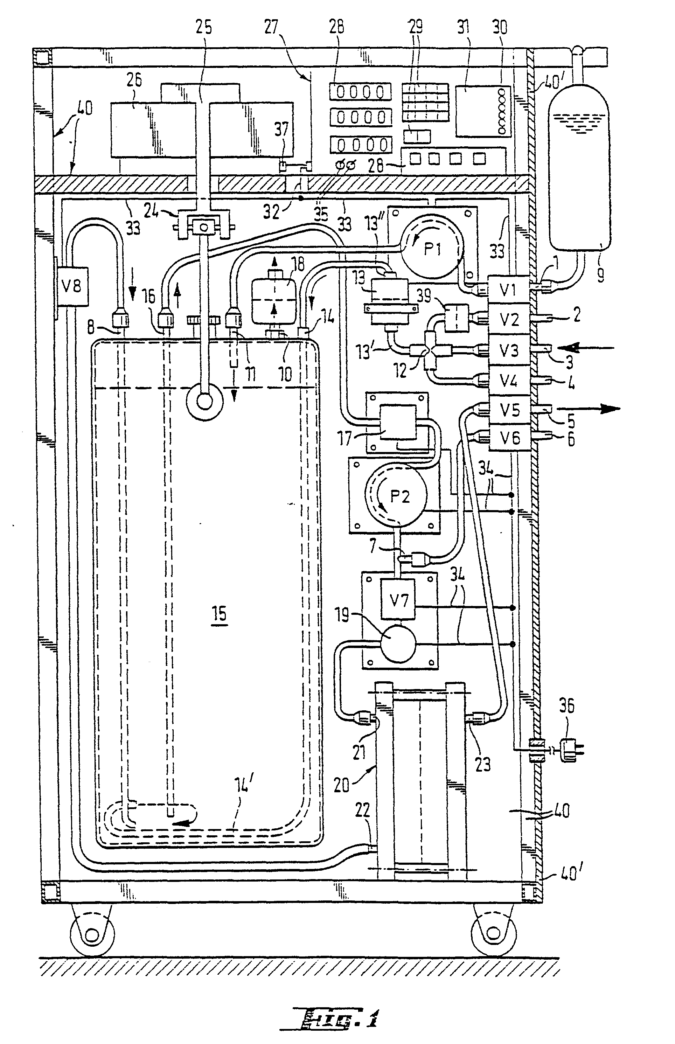

Figur 1 das portable Gerätegestell mit den darin angeordneten einzelnen Geräteelementen in vereinfachter Darstellung und

Figur 2 eine auf der Gerätefrontseite angeordnete Schaltplanfolie.

Die noch näher zu beschreibenden Einzelgeräteteile sind auf ein mit Transportrollen ausgerüstetes Gerätegestell 40 montiert. Das Gestell 40 ist zumindest stellenweise durch eine Verkleidung 40' geschlossen.

Das Gerät besteht im wesentlichen aus den Absperrventilen V1 bis V8, die einlaßseitig und auslaßseitig mit selbstsperrenden Anschlüssen zur Aufnahme von Schläuchen ausgestattet sind, den Pumpen P1 und P2, einem Leitfähigkeitsmesser 17, einem Druckmesser 19, einem Ultrafilter 20, mindestens drei Sterilfiltern 13, 39, 18, einem Mischbehälter 15, einer Waage 26 und einem Steuer- und Regelgerät 27.

Im dargestellten Ausführungsbeispiel ist der Mischbehälter 15 über zwei Kreuzgelenke 24 und ein Joch 25 freihängend und pendelnd und die oberschalige Waage 26 belastend, aufgehängt. Bei Anordnung der Waage 26 am Gestellboden kann der Mischbehälter 15 natürlich auch auf der Waage 26 stehen. Anstelle der Waage 26 können auch andere herkömmliche Kraftaufnehmer als gravimetrischer Meßwertgeber dienen.

Alle übrigen Geräteteile sind mittelbar oder unmittelbar am Gestell 40 durch Verschraubungen oder dergleichen festgelegt.

Der Konzentratvorratsbehälter 9 für das Elektrolytkonzentrat hängt z. B. an einem Galgen des Gestell 40 und der Behälterauslaß mündet im Anschluß 1 des Absperrventils V1. Eine elektromotorisch angetriebene Pumpe P1 ist zwischen dem Konzentratvorratsbehälter 9 und Ventil 1 angeordnet und fördert Konzentrat in den Einlaß 11 des Mischbehälters 15. An den Einlaß 3 des Absperrventils V3 ist über einen Schlauch die Verbindung mit einem nicht dargestellten Reinstwasserreservoir vorgesehen. Der Ausgang des Ventils V3 geht in einen kreuzförmigen Stromteiler 12 über, dessen zwei Auslässe in den beiden Absperrventilen V2 bzw. dessen Flüssigkeitsanschluß 2 und im Absperrventil V4 bzw. Flüssigkeitsanschluß 4 münden. Der dritte Auslaß des Stromteilers 12 mündet auf der Einlaßseite 13' eines ersten Sterilfilters 13 von 0,2 μm Porengröße, der im Reinstwasser vorhandene Keime zurückhält und der Auslaß 13" des Sterilfilters 13 mündet in einem weiteren Einlaß 14 des Mischbehälters 15, der zur besseren Durchmischung auf dem Behälterboden kreis- oder spiralförmig mit einer Düse endet. Zwischen dem Absperrventil V2 und dem Stromteiler 12 ist ein zweiter Sterilfilter 39 mit 0,2 μm Porengröße angeordnet.

Ein Auslaß 16 des Mischbehälters 15 mündet in einem elektrischen Leitfähigkeitsmesser 17, der die elektrische Leitfähigkeit der Flüssigkeit im Mischbehälter 15 mißt, die abhängig von der aktuellen Konzentration der Flüssigkeit ist. Der Ausgang des Konzentrationsmessers 17 mündet in einem T-förmigen Stromteiler 7, der mit dem Flüssigkeitsanschluß 6 und dem Absperrventil V6 einen Bypass bildet. Weiterhin ist der Stromteiler 7 mit einem weiteren Absperrventil V7 und dem Einlaß 21 eines Ultrafilters 20 mit einer Pyrogene zurückhaltenden Membran mit einem Cut off von ≤ 20 000 Daltons verbunden. Zwischen dem Einlaß 21 des Ultrafilters 20 und dem Auslaß 16 des Mischbehälters 15 sind weiterhin der bereits erwähnte Leitfähigkeitsmesser 17, eine Förderpumpe P2, der Stromteiler 7, das Absperrventil V7 und ein Flüssigkeitsdruckmesser 19 angeordnet. Je nach Ausführungsform werden diese Bauteile mittelbar oder unmittelbar von der Flüssigkeit durchströmt.

Der trübseitige Auslaß 22 des Ultrafilters 20 mündet unter Zwischenschaltung eines weiteren Absperrventiles V8 in einem weiteren Einlaß 8 des Mischbehälters 15 gleichfalls in einer Kreisform am Behälterboden. Der reinseitige Auslaß 23 des Ultrafilters 20 mündet in dem Absperrventil V5 und dem Flüssigkeitsanschluß 5. Dieser Anschluß 5 dient zur direkten Weiterleitung der aufbereiteten Infusionslösung zur Abfüllung in einen Behälter zur Zwischenlagerung oder direkt an den oder die Verbraucher, das sind unter Zwischenschaltung der Dialysemaschinen oder Haemofiltrationsmaschinen die nierenerkrankten Patienten.

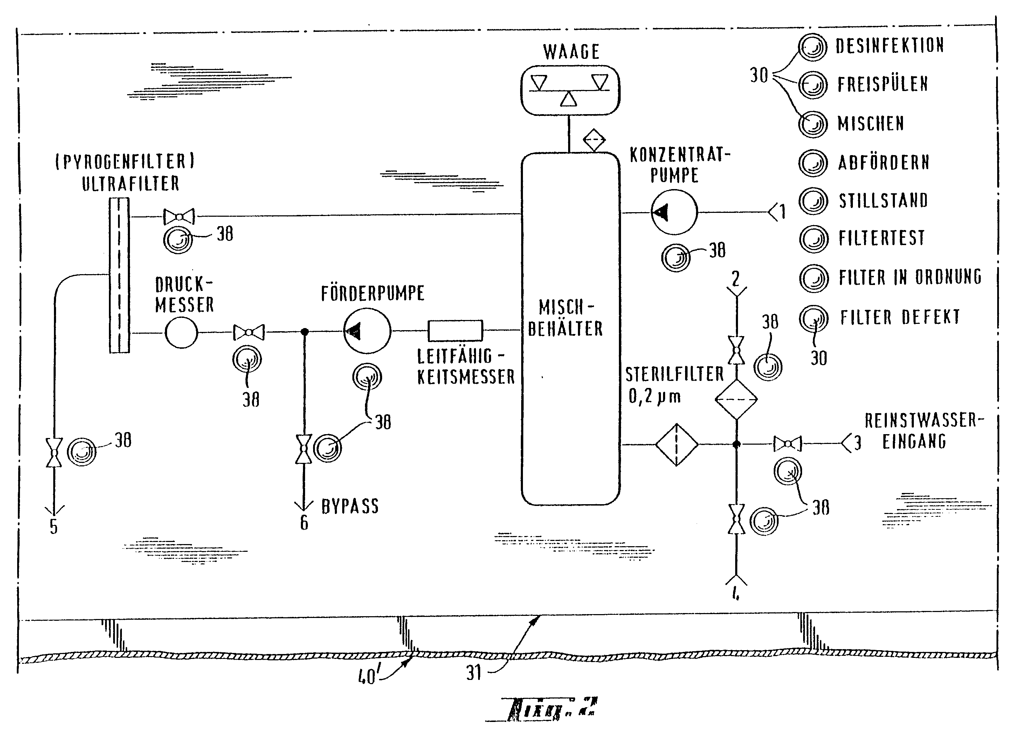

Das elektrische Steuer- und Regelgerät 27 weist mehrere alpha-numerische Anzeigen 28 in digitaler Form sowie eine Eingabetastatur 29, Kontrollampen 30 und eine Schaltplanfolie 31 oder Aufdruck auf, die in Fig. 2 näher erläutert ist. Die elektrischen Geräteteile der Waage 26, des Steuer- und Regelgerätes 27, der beiden Pumpen P1 und P2, der Absperrventile V1 bis V8, des Leitfähigkeitsmessers 17 und des Druckmessers 19 kontaktieren untereinander mit Hilfe elektrischer Leitungen 32, 33 und 34 und sind über einen Netzanschlußstecker 36 an das Stromnetz angeschlossen bzw. anschließbar. Das Steuer- und Regelgerät 27 weist weiterhin mehrere Funktionsschalter 35 auf. Die dargestellte handelsübliche Waage 26 ist mit einem Mikroprozessor ausgestattet und ist über einen Datenausgang 37 mit dem Steuer- und Regelgerät 27 kommunizierend verbunden.

Das Gerät kann permanent mit V3 an einen Reinstwassereingang angeschlossen werden.

Vor Inbetriebnahme des Gerätes zur Mischung der Infusionslösung muß dieses sterilisiert und vom Sterilisierungsmittel freigespült werden. Hierbei kann eine chemische Sterilisation mit Formalin oder eine Heißsterilisation erfolgen. Zu diesem Zweck wird das chemische Sterilisationsmittel in konzentrierter Form in den Flüssigkeitsanschluß 1 eingeleitet, wobei alle anderen Ventile geschlossen sind. Das konzentrierte Sterilisationsmittel wird gemäß dem eingegebenen Mischungsverhältnis mit Reinstwasser im Mischbehälter verdünnt.

V1 wird mit V2 und V4 wird mit V5 mittels eines kurzen Schlauchverbindungsstückes verbunden. So ist das gesamte Leitungssystem in sich geschlossen und sämtliche flüssigkeitführenden Baugruppen können durch entsprechende Schaltung der Ventile und Pumpen chemisch desinfiziert werden. Eine im Gerät eingebaute Uhr zeigt die Dauer der Desinfektion an.

Nach vollendeter Desinfektion wird das Desinfektionsmittel über den Bypass 6 verworfen. Das gesamte Leitungssystem wird mit Reinstwasser von Desinfektionsmittelresten durch entsprechende Schaltung der Ventile freigespült. Die Anzahl der Spülzyklen wird angezeigt.

Der Mischbehälter 15 faßt etwa 50 l Flüssigkeit. Zur Herstellung von einer bestimmten Menge Infusionslösung wird 1. das gewünschte volumetrische Mischungsverhältnis (Konzentrat/Reinstwasser), 2. das spez. Gewicht des Konzentrats (das spez. Gewicht des Reinstwasser wird vom Rechner automatisch mit 1 gr/ml angesetzt) und 3. die Gesamtmenge der herzustellenden Lösung in das Gerät eingegeben.

Daraufhin wird evtl. im Gerät befindliche Flüssigkeit über den Bypass 6 entleert.

Da das Mischungsverhältnis, wie in der Dialyse üblich, volumetrisch eingegeben wurde, wird vom Rechner mit Hilfe des spez. Gewichtes des Konzentrates automatisch das gravimetrische Mischungsverhältnis berechnet. Die berechnete Konzentratmenge wird von der Pumpe P1 in den leeren Mischbehälter 15 dosiert. Bei Erreichen des Sollgewichtes stoppt die Pumpe P1. Im Ruhezustand wird das Konzentratgewicht durch eine Wägung exakt bestimmt. Bestehen Differenzen zwischen Soll- und Istgewicht des Konzentrates, so wird die zu fördernde Reinstwassermenge entsprechend dem Ist-Konzentratgewicht neu berechnet. Auf diese Art ist eine exakte Einstellung des Mischungsverhältnisses gewährleistet. Dann öffnet V3 und das Reinstwasser strömt via V3, Sterilfilter 13 durch ein Tauchrohr 14' in den Mischbehälter 15. Das Tauchrohr 14' ist so ausgebildet, daß im Mischgefäß 15 eine kreisförmige Verwirbelung und damit eine homogene Vermischung der Lösung erfolgt. Ist die berechnete Menge in das Mischgefäß 15 geflossen, schließt V3. Dann startet die Pumpe P2 und V8 wird geöffnet (alle anderen Ventile sind geschlossen). Hierdurch wird eine zusätzliche Vermischung der Infusionslösung erreicht, die Trübseite des Ultrafilters 20 wird von Luft befreit, und die Oberfläche des Ultrafilters 20 wird gereinigt. Damit ist das Mischprogramm beendet.

Jetzt kann über die Eingabetastatur 29 die Infusionslösungsmenge eingegeben werden, die z. B. in einen Kontrollbeutel abgefüllt werden soll. Diese Menge wird aus dem Mischbehälter 15 durch die Pumpe P2 abgefördert, mit einem Leitfähigkeitsmesser 27 auf exakte Konzentration überprüft und durch das Pyrogenfilter entsprechend und durch V5 in ein Behältnis abgefüllt. Bei Abweichung der Leitfähigkeit vom Sollwert schließt V5 und die Lösung wird über den Bypass verworfen. Die verworfene Menge wird zur abgefüllten Menge nicht hinzugezählt.

Zum Schutz vor Verkeimung während einer längeren Entnahmepause (Nichtentnahme von Infusionslösung) besitzt das Gerät ein weiteres spezielles Programm. Zu diesem Zweck wird V4 mit V5 mittels einer Schlauchverbindung verbunden. Pumpe P2 hält einen Flüssigkeitsstrom, der vom Mischbehälter 15 über Pumpe P2, Pyrogenfilter 20, V5, V4, Sterilfilter 13 zurück in den Mischbehälter 15 rezirkuliert, aufrecht. Dies bedeutet, daß der reinseitige Auslaß 23, V5 des Pyrogene zurückhaltenden Ultrafilters 20 mit dem Einlaß 14 des Mischbehälters 15, vorzugsweise mit dem vorgelagerten Einlaß V4, 13' des Sterilfilters 13 verbindbar ist und durch die Förderpumpe P2 die aufbereitete Infusionslösung in einem geräteinternen Rezirkulationskreislauf vom Mischbehälter 15 über den Ultrafilter 20, Sterilfilter 13 und zurück in den Mischbehälter 15 bewegbar ist. Der trübseitige Auslaß 22 des Ultrafilters 20 ist dabei in den von der Förderpumpe P2 aufrechterhaltenen Rezirkulationskreislauf im Nebenschluß Ultrafilter/Mischbehälter einbezogen.

Zur sterilen Belüftung und Entlüftung des Mischbehälters 15 ist außerdem ein Belüftungsfilter 18 vorgesehen, der auf einen weiteren Auslaß 10 des Mischbehälters 15 aufgesetzt ist.

Die Bedienungs- und Anzeigeelemente des Steuer- und Regelgerätes 27 sind in die Verkleidung integriert ebenso die Schaltplanfolie 31, die durch elektrische Lampen den aktuellen Schaltzustand des Gerätes in den einzelnen Schaltphasen optisch anzeigt und in Fig. 2 näher dargestellt ist. Der Schaltplan ist auf einer Folie aufgedruckt bzw. direkt auf der Geräteverkleidung aufgezeichnet. Unmittelbar neben den Symbolen für die vom Steuer- und Regelgerät 27 beeinflußbaren elektrischen Geräteteilen, das sind die beiden Pumpen P1 und P2 und die Absperrventile V1 bis V8 sind Gehäusedurchbrechungen angeordnet, in denen kleine Signallampen befestigt sind, die das Steuer- und Regelgerät 27 kontaktieren und den jeweiligen Schaltzustand durch ihren ein- bzw. ausgeschalteten Zustand anzeigen. In Fig. 2 sind diese Signallampen einheitlich mit 38, 30 bezeichnet. Das Bedienungspersonal hat aufgrund dieser vorteilhaften Ausgestaltung die Möglichkeit, den aktuellen Programmablauf, die Steuerung der Ventile und der anderen elektrischen Baueinheiten optisch zu verfolgen und eventuelle Fehler oder Ausfälle sofort zu erkennen.

**Patentansprüche**

1. Gerät zur Aufbereitung medizinischer Infusionslösungen, insbesondere für die Verwendung derselben zur Behandlung der Urämie mit der Hämofiltration oder der Peritonealdialyse, gekennzeichnet durch die Kombination folgender unmittelbar oder mittelbar verbundener Baueinheiten :

a) ein Konzentratvorratsbehälter (9) für das Infusionskonzentrat mündet·mit seinem Auslaß in einen Mischbehälter (15),

· b) zwischen Mischbehälter (15) und Konzentratvorratsbehälter (9) ist eine elektromotorisch angetriebene Konzentratpumpe (P1) angeordnet, welche Konzentrat in den Mischbehälter (15) fördert,

c) mehrere Flüssigkeitsanschlüsse (1 bis 6) sind in Kombination mit Absperrventilen (V1 bis V6) angeordnet, die elektrisch bewegliche Absperrorgane aufweisen,

d) ein Flüssigkeitsanschluß (3) davon ist ein Reinstwassereingang für ein durch Umkehrosmose oder durch andere Verfahren hergestelltes Reinstwasser aus einem Reinstwasserreservoir, wobei der Flüssigkeitsanschluß (3) in den Eingang (13') eines Keime zurückhaltenden Sterilfilters (13) mit 0,2 $\mu$m Porengröße und der Ausgang (13") des Sterilfilters (13) in einen weiteren Einlaß (14) des Mischbehälters (15) mündet,

e) ein Auslaß (16) des Mischbehälters (15) mündet im Einlaß (21) eines am Gestell (40) festgelegten oder abgestützten, Pyrogene zurückhaltenden Ultrafilters (20) mit einem Cut off von $\leqslant$ 20 000 Daltons und der trübseitige Auslaß (22) des Ultrafilters (20) mündet in einen weiteren Einlaß (8) des Mischbehälters (15),

f) zwischen dem Einlaß (21) des Ultrafilters (20) und dem Auslaß (16) des Mischbehälters (15) ist ein elektrischer Flüssigkeitsdruckmesser (19), eine elektromotorisch angetriebene Förderpumpe (P2) und ein elektrischer Leitfähigkeitsmesser (17) für die Messung der aktuellen Konzentration der Infusionslösung angeordnet,

g) der reinseitige Auslaß (23) des Ultrafilters (20) mündet in einem Zapf- und Absperrventil (V5),

h) der Mischbehälter (15) belastet stehend oder hängend einen am Gestell (40) festgelegten Kraftmesser oder eine Waage (26),

i) ein elektrisches Steuer- und Regelgerät (27) für die Bilanzierung des Mischungsverhältnisses von Konzentrat und Reinstwasser, ausgestattet mit einer manuellen Eingabetastatur

4

(29), mehreren Funktionsschaltern (35), Kontrollampen (30) und alpha-numerischen Anzeigen (28) kontaktiert die elektrischen Bauteile des Kraftmessers oder Waage (26), der beiden Pumpen (P1, P2) der Absperr- und Zapfventile (V1 bis V8), des Druckmessers (19) und des Konzentrationsmesser (17),

j) zum Schutz vor Verkeimung der Flüssigkeit im Gerät während einer längeren Entnahmepause (Nichtentnahme von Infusionslösung) ist der reinseitige Auslaß (23, V5) des Pyrogene zurückhaltenden Ultrafilters (20) mit dem Einlaß (14) des Mischbehälters (15), vorzugsweise mit dem vorgelagerten Einlaß (V4, 13') des Sterilfilters (13) verbindbar und durch die Förderpumpe (P2) ist die aufbereitete Infusionslösung in einem geräteinternen Rezirkulationskreislauf vom Mischbehälter (15) über den Ultrafilter (20), Sterilfilter (13) und zurück in den Mischbehälter (15) bewegbar.

2. Gerät nach Anspruch 1, dadurch gekennzeichnet, daß der trübseitige Auslaß (22) des Ultrafilters (20) in den von der Förderpumpe (P2) aufrechterhaltenen Rezirkulationskreislauf im Nebenschluß Ultrafilter (20)/Mischbehälter (15) einbezogen ist.

3. Gerät nach Anspruch 1 und 2, dadurch gekennzeichnet, daß zwischen dem Ultrafilter (20) und dem Mischbehälter (15) zusätzliche Absperrventile (V6, V7, V8) angeordnet sind.

4. Gerät nach Anspruch 1 bis 3, dadurch gekennzeichnet, daß zwischen dem Ultrafilter (20), der Förderpumpe (P2) und dem Mischbehälter (15) ein Bypass (7) mit einem Absperrventil (V6) angeordnet ist.

5. Gerät nach Anspruch 1 bis 4, dadurch gekennzeichnet, daß zwischen dem Sterilfilter (13) und dem Absperrventil (V3) für den Anschluß (3) des Reinstwassers ein kreuzförmiger Stromteiler (12) angeordnet ist, dessen Auslässe in weiteren Absperrventilen und in mindestens einem weiteren Sterilfilter (39) von 0,2 μm Porengröße münden.

6. Gerät nach Anspruch 1 bis 5, dadurch gekennzeichnet, daß die Absperrventile (V1 bis V8) elektromagnetisch gesteuerte Membranventile sind.

7. Gerät nach Anspruch 1 bis 6, dadurch gekennzeichnet, daß der Einlaß (14) des Mischbehälters (15) für das Reinstwasser als Tauchrohr (14') mit einem am Behälterboden endenden, die Durchmischung fördernden gebogenen Rohrende ausgebildet ist.

8. Gerät nach Anspruch 1 bis 7, dadurch gekennzeichnet, daß sämtliche Flüssigkeit führenden Leitungen und Baugruppen durch fluiden Kurzschluß einzelner Ein-/Auslässe (1 bis 6) der Ventile (V1 bis V6) untereinander und deren selektive Absperrung zu einem von einem Sterilisationsmittel durchspülten geschlossenen Kreislauf verbindbar sind.

9. Gerät nach Anspruch 1, dadurch gekennzeichnet, daß der Mischbehälter (15) in einem weiteren Auslaß (10) einen Filter (18) zur sterilen Be- und Entlüftung trägt.

10. Gerät nach Anspruch 1 bis 9, dadurch gekennzeichnet, daß die Gestellverkleidung (40') Durchbrechungen in Größe von zur Geräteaußenseite weisenden Lampenfassungen für Signallampen aufweist und in den Durchbrechungen Signallampen (38, 30) angeordnet sind und einem Teil der Signallampen (38) unmittelbar ein auf der Gehäuseverkleidung (40') befestigter Schaltsymbolträger (31) für die elektrisch gesteuerten Ventile (V1 bis V8) und die beiden Pumpen (P1 und P2) zugeordnet ist.

11. Gerät nach Anspruch 1 bis 9, dadurch gekennzeichnet, daß sämtliche Baueinheiten mittelbar oder unmittelbar an einem gemeinsamen Gerätegestell (40) festgelegt sind.

## Claims

1. Apparatus for preparing medical infusion solutions, especially for use of said solutions in the treatment of uremia by hemofiltration or peritoneal dialysis, characterized by the combination of the following directly or indirectly connected constructional units :

a) a concentrate reserve container (9) for the infusion concentrate, the container outlet opening into a mixing tank (15) ;

b) arranged between said mixing tank (15) and concentrate reserve container (9) is an electromotor-driven concentrate pump (P1) which conveys concentrate into the mixing tank (15) ;

c) a plurality of liquid coupling means (1 to 6) are arranged in combination with shutoff valves (V1 to V6) which have electrically movable blockage and control elements ;

d) a liquid coupling means (3) of said plurality is a high purity water inlet for high purity water generated from a high purity water reservoir by reverse osmosis or by other methods, with the liquid coupling means (3) opening into the inlet (13') of a microbe-retentive, 0.2 μm pore size sterilizing filter (13), and the outlet (13") of the sterilizing filter (13) opening into an additional port (14) of the mixing tank (15) ;

e) an outlet (16) of the mixing tank (15) opens into the inlet (21) of a pyrogen-retentive ultrafilter (20) having a cutoff of ≤ 20 000 daltons and located or supported on the apparatus frame (40) ; and the upstream outlet (22) of said ultrafilter (20) opens into an additional port (8) of the mixing tank (15) ;

2. Apparatus as in claim 1, wherein the upstream outlet (22) of the ultrafilter (20) is included in the shunt path of the ultrafilter (20) and mixing tank (15) in the recirculation circuit maintained by the conveying pump (P2).

3. Apparatus as in claims 1 and 2, wherein additional shutoff valves (V6, V7, V8) are arranged between the ultrafilter (20) and the mixing tank (15).

4. Apparatus as in claims 1 to 3, wherein a bypass (7) with a shutoff valve (V6) is disposed among the ultrafilter (20), the conveying pump (P2) and the mixing tank (15).

5. Apparatus as in claims 1 to 4, wherein a cross-shaped flow divider (12) is arranged between the sterilizing filter (13) and the shutoff valve (V3) for the coupling means (3) of the high purity water, the outlets of said divider opening into additional shutoff valves and into at least one additional 0.2 μm pore size sterilizing filter (39).

6. Apparatus as in claims 1 to 5, wherein the shutoff valves (V1 to V8) are electromagnetically controlled diaphragm valves.

7. Apparatus as in claims 1 to 6, wherein the inlet (14) of the mixing tank (15) for the high purity water is formed as an immersion pipe (14') with the bent end portion ending at the tank base and promoting intermixture.

8. Apparatus as in claims 1 to 7, wherein all liquid-conducting ducts and structural components are connectable to one another by fluid short-circuit of individual inlets and outlets (1 to 6) of the valves (V1 to V6) and by their selective blocking in order to form a closed circuit thoroughly flushed through by a sterilant.

9. Apparatus as in claim 1 wherein the mixing tank (15) holds in an additional outlet (10) a filter (18) for sterile venting and aeration.

10. Apparatus as in claims 1 to 9, wherein the frame casing (40') has openings of the size of lamp sockets, facing the outside of the apparatus, for signal lamps, and in said openings signal lamps (38, 30) are positioned and a means bearing flow circuit symbols (31) and directly attached to the apparatus casing (40') for the electrically controlled valves (V1 to V8) and both pumps (P1 and P2) is associated with part of the signal lamps (38).

11. Apparatus as in claims 1 to 9, wherein all constructional units are located indirectly or directly on a common apparatus frame (40).

**Revendications**

1. Appareil pour la préparation de solutions médicales pour infusion, en particulier pour l'utilisation de ces solutions pour traiter l'urémie par l'hémofiltration ou la dialyse péritonéale, appareil caractérisé par la combinaison des modules suivants, reliés directement ou indirectement :
    a) un réservoir (9) de stockage du concentré pour infusion débouche par sa sortie dans un réservoir (15) de mélange,
    b) entre le réservoir (15) de mélange et le réservoir (9) de stockage de concentré, est montée une pompe (P1) à concentré, entraînée par un moteur électrique et qui envoie le concentré dans le réservoir (15) de mélange,
    c) plusieurs tubulures (1 à 6) à liquide sont montées en combinaison avec des soupapes (V1 à V6) d'arrêt, qui présentent des organes d'obturation électriquement manœuvrables,
    d) une tubulure (3) à liquide, reliée à l'une de ses soupapes, constitue une entrée d'une eau très pure produite par osmose inverse ou par un autre procédé, et provenant d'un réservoir d'eau très pure, la tubulure (3) débouchant dans l'admission (13') d'un filtre (13), ayant des pores de 0,2 μm de grandeur, pour stérilisation par retenue de germes, la sortie (13") du filtre (13) de stérilisation débouchant dans une autre entrée (14) du réservoir (15) du mélange,
    e) une sortie (16) du réservoir (15) de mélange débouche dans l'admission (21) d'un ultrafiltre (20) ayant un pouvoir de coupure à ≤ 20 000 daltons et retenant les pyrogènes, filtre fixé à ou supporté par le châssis (40), et la sortie (22), côté trouble, de l'utrafiltre (20) débouche dans une autre admission (8) du réservoir (15) de mélange,
    f) entre l'admission (21) de l'ultrafiltre (20) et la sortie (16) du réservoir (15) de mélange, sont montés un appareil (19) électrique de mesure de pression de liquide, une pompe (P2) de circulation entraînée par un moteur et un appareil (17) de mesure de la conductibilité électrique, pour mesurer la concentration actuelle de la solution pour infusion,
    g) la sortie (23), côté épuré, de l'ultrafiltre (20) débouche dans une soupape (V5) de fourniture et obturation,
    h) le réservoir (15) de mélange repose, de manière à le charger, sur ou est suspendu à un appareil de mesure ou une balance (26) fixé(e) au châssis (40),
    i) un dispositif (27) électrique de commande et de régulation pour établir le bilan du rapport de mélange de concentré et d'eau très pure, dispositif équipé d'un clavier (29) manuel d'introduction de données, de plusieurs interrupteurs (35) de fonctions, de lampes (30) témoins et d'affichages (28) alphanumériques est en contact avec les composants électriques de l'appareil de mesure de force ou balance (26), les deux pompes (P1, P2) des soupapes (V1 à V8) d'arrêt et de distribution, de l'appareil (19) de mesure de pression et de l'appareil (17) de mesure de concentration,
    j) pour protéger contre une infection par des germes ou contre la propagation de germes dans le liquide se trouvant dans l'appareil pendant un assez long repos sans soutirage (sans prélèvement de la solution pour infusion), la sortie (23, V5) du côté épuré de l'ultrafiltre (20) retenant les pyrogènes peut être reliée à l'admission (14) du réservoir (15) de mélange, avantageusement avec l'admission (V4, 13'), reliée en amont, du filtre (13) de stérilisation, et la pompe (P2) de circulation peut déplacer la solution préparée pour infusion et lui faire parcourir un circuit de recyclage interne à l'appareil, à partir du réservoir (15) de mélange en passant par l'ultrafiltre (20) et le filtre (13) de stérilisation pour revenir dans le réservoir (15) de mélange.

2. Appareil selon la revendication 1, caractérisé en ce que la sortie (22), côté trouble, de l'ultrafiltre (20) est intégrée, dans un circuit de dérivation ultrafiltre (20)/réservoir (15) de mélange, au circuit de recyclage entretenu par la pompe (P2) de circulation.

3. Appareil selon les revendications 1 et 2, caractérisé en ce que des soupapes (V6, V7, V8) supplémentaires d'arrêt sont montées entre l'ultrafiltre (20) et le réservoir (15) de mélange.

4. Appareil selon l'une des revendications 1 à 3, caractérisé en ce qu'un bipasse (7), équipé d'une soupape (V6) d'arrêt, est monté entre l'ultrafiltre (20), la pompe (P2) de circulation et le réservoir (15) de mélange.

5. Appareil selon l'une des revendications 1 à 4, caractérisé en ce que, entre le filtre (13) de stérilisation et la soupape (V3) d'arrêt de la tubulure (3) d'eau très pure, est monté un diviseur (12) cruciforme de débit dont les sorties débouchent dans d'autres soupapes d'arrêt et dans au moins un autre filtre (39) de stérilisation ayant des pores de 0,2 µm.

6. Appareil selon l'une des revendications 1 à 5, caractérisé en ce que les soupapes (V1 à V8) d'arrêt sont des soupapes à membrane à commande électromagnétique.

7. Appareil selon l'une des revendications 1 à 6, caractérisé en ce que l'admission (14) d'eau très pure dans le réservoir (15) de mélange est réalisée sous forme d'un tube (14') plongeur comportant une extrémité se terminant au fond du réservoir et repliée de manière à accélérer le processus de mélange.

8. Appareil selon l'une des revendications 1 à 7, caractérisé en ce que tous les conduits et composants conduisant du liquide peuvent être reliés les uns aux autres par court-circuitage par du fluide des diverses admissions/sorties (1 à 6) des soupapes (V1 à V6) et par leur obturation sélective pour former un circuit fermé parcouru par un courant de balayage par un agent de stérilisation.

9. Appareil selon la revendication 1, caractérisé en ce que le réservoir (15) de mélange porte, dans une autre sortie (10), un filtre (18) pour assurer de façon stérile une entrée et une sortie d'air.

10. Appareil selon l'une des revendications 1 à 9, caractérisé en ce que le boîtier (40') de carrossage présente des découpures dont la grandeur correspond à des douilles pour lampes témoins apparaissant du côté extérieur de l'appareil, en ce que des lampes témoins (38, 30) sont disposées dans les découpures et en ce qu'un support (31) de symboles de connexion, fixé au boîtier (40') de carrossage, est directement associé à une partie des lampes témoins (38) correspondant aux soupapes (V1 à V8) à commande électrique et aux deux pompes (P1 et P2).

11. Appareil selon l'une des revendications 1 à 9, caractérisé en ce que tous les modules et composants sont fixés, directement ou indirectement, à un châssis (40) commun pour tout l'appareil.

**FIG. 1**

WAAGE

(PYROGENFILTER)
ULTRAFILTER

DRUCK-
MESSER

FÖRDERPUMPE

LEITFÄHIG-
KEITSMESSER

MISCH-
BEHÄLTER

KONZENTRAT-
PUMPE

STERILFILTER
0,2 μm

REINSTWASSER-
EINGANG

BYPASS

38 38 38 38 38 38

30 DESINFEKTION

FREISPÜLEN

MISCHEN

ABFÖRDERN

STILLSTAND

FILTERTEST

FILTER IN ORDNUNG

FILTER DEFEKT

30

1 2 3 4 5 6

40' 31

Fig. 2

0 121 085